# EUROPEAN PATENT APPLICATION

(11) **EP 4 385 449 A1**
(43) Date of publication of application: **19.06.2024**
(21) Application number: 22315330.5
(22) Date of filing: 16.12.2022
(51) Int. Cl.: A61B 90/00

(54) **SYSTEM FOR POSITIONING A MODULE**

(71) Applicant: Caranx Medical SAS, 75013 Paris (FR)
(72) Inventor: Berthet-Rayne, Pierre, 06800 Cagnes-sur-Mer (FR); Bechar, ikhlef, 06410 Biot (FR); Cerruti, Giulio, 06700 Saint-Laurent-Du-Var (FR)
(74) Representative: Hepp Wenger Ryffel AG

(57) **Abstract**

The invention relates to a system (200) for positioning a medical device with respect to at least one target zone (40) of a patient (70). The system comprises a mount (30) for a medical module (31), and a positioning device (20) configured to move the mount (30) for a medical module (31) with respect to the at least one target zone (40). The system further comprises an imaging means (10), configured to at least obtain imaging data of at least one landmark (50, 51, 52) arranged in a predefined relationship to the at least one target zone (40). The system further comprises a computational system (80). The computational system (80) comprises an input interface (81) for receiving said imaging data. The computational system (80) further comprises an output interface (82) for outputting data for controlling the movement of the positioning device (20). The computational system (80) is configured to determine a position of the at least one landmark (50, 51, 52) based on the imaging data. The computational system (80) is further configured to control the position of the positioning device (20) with respect to the at least one target zone (40) based on the determined position of the at least one landmark (50, 51, 52).

## Description

The invention relates to a system for positioning a medical device with respect to at least one target zone, a computational system for determining position data based on imaging data, a method for positioning a module with respect to a target zone, a computer program product for executing method steps for positioning a module, and a non-transient computer readable storage medium storing the computer program product, according to the preamble of the independent claims.

Methods that allow to obtain position information of patients are known.

US 9,789,338 B1 discloses a patient monitoring system for monitoring a patient undergoing radiotherapy comprising a projector operable to project a pattern of light onto a patient undergoing radiation treatment. A patient restraint is operable to restrain the patient relative to a treatment apparatus. An image detector is operable to obtain images of the patient, and a model generation module is operable to process images of the patient obtained by the image detector to generate a model of the surface of a portion of the patient. At least a portion of the patient restraint is colored and the model generation module is inhibited from generating a model of the colored portion of the patient restraint.

US 2,019,134,426 A1 discloses images obtained by a camera system arranged to obtain images of a patient undergoing radio-therapy that are processed by a modelling unit which generates a model of the surface of a patient being monitored. Additionally the patient monitoring system processes image data not utilized to generate a model of the surface of a patient being monitored to determine further information concerning the treatment of the patient. Such additional data can comprise data identifying the relative location of the patient and a treatment apparatus. This can be facilitated by providing a number or retro-reflective markers on a treatment apparatus and a mechanical couch used to position the patient relative to the treatment apparatus and monitoring the presence and location of the markers in the portions of the images obtained by the stereoscopic camera.

The paper entitled "AutomatedMarker Localization in the Planning Phase of Robotic Neurosurgery," in IEEE Access, vol. 5, pp. 12265-12274, 2017, doi: 10.1109/ACCESS.2017.2718621, discloses that accurate patient registration is a critical issue in medical image-guided interventions. The neurosurgical robotic system RObotic Neuro-NAvigation (RONNA) uses four retro-reflective spheres, on a marker attached to the patient's cranial bone, for patient registration in physical and image space. In this paper, the algorithm for automatic localization of spherical fiducials in CT scans is presented and clinically evaluated. The developed localization algorithm combines machine vision algorithms, biomedical image filtration methods, and mathematical estimation methods. The performance of the localization algorithm was evaluated in comparison with four skilled human operators. The developed algorithm provides machine vision-based patient localization for the neurosurgical clinical application of the robotic system RONNA.

US 8,047,991 B2 discloses that orientation is automatically identified in medical diagnostic ultrasound image. An area or volume based process, such as region shrinking or using locations associated with flow or tissue structure, determines a direction or orientation. Real-time imaging in B-mode and/or flow mode may be enhanced based on the direction or orientation.

US 9,792,408 B2 discloses that the presence or absence of objects tagged with transponders may be determined in an environment in which medical procedures are performed via an interrogation and detection system which includes a controller and a plurality of antennas positioned along a patient support structure. The antennas may be positioned along an operating table, bed, mattress or pad, sheet, or may be positioned on a surgical drape, or shade. A wireless physiological condition monitor may detect patient physiological conditions and wirelessly transmit signals indicative of such.

EP 1569576 B1 discloses a method of determining a surgical patient's pelvic position and inputting that position into a computer via a tracking system, suitable for use in navigating partial or total hip replacement surgery. According to the method, the patient is first aligned with anatomical reference points in relation to corresponding locating features on a patient positioner. The positions of index features on the patient positioner are then acquired via a tracking system. Based on the positions of the index features and their known relationship to the locating features, the locations of the anatomical reference features are calculated and a pelvic plane is defined therefrom.

EP 3254621 B1 discloses a calibrator for a 3D image, a surgical positioning system, and a positioning method. The calibrator for the 3D image includes a calibrator plane and a calibrator handle. The calibrator plane is flat or arc-shaped, and at least four marked points to be identified by a 3D imaging device are arranged on the calibrator plane. One end of the calibrator handle is fixedly connected to the calibrator plane, and the other end of the calibrator is provided with a connector for connecting to a surgical robotic arm.

EP 1858430 A1 discloses a method, apparatus and computer program code for automatically planning at least a part of a surgical procedure to be carried out on a body part of a patient. A virtual model of the body part is provided, in which the model has data associated with it representing at least a part of a planned surgical procedure. The virtual model is then morphed to the body part using data derived from the patient's real body part thereby also adapting the part of the planned surgical procedure to reflect the anatomy of the patient's real body part.

US 2004177449 A1 discloses an adjustable mattress and pillow system and related methods in which a sensing mat positioned on the top face of a mattress affects microprocessor-controlled optimization of the contour of the mattress and a pillow based on a user's position. In one embodiment, the mattress and pillow system provides real time contour optimization through use of a variety of sensing techniques that make the system useful in environments such as hospital critical care facilities.

It is an object of the invention to overcome the disadvantages of the prior art, in particular, to provide a more precise, more repeatable, less error prone (especially less human error prone), and/or more flexible positioning system for positioning a medical device on a patient. Further, it is an object of the invention to provide more flexible, adaptable and/or robust markers and monitoring thereof.

This object is solved by the systems, methods, products, and devices defined in the independent patent claims. Further embodiments result from the dependent patent claims.

A system according to the invention comprises a mount for a medical module, and a positioning device that is configured to move the mount for the medical module with respect to the at least one target zone. The system further comprises an imaging means, which is configured to at least obtain imaging data of at least one landmark arranged in a predefined relationship to the at least one target zone. The predefined relationship may refer to a position and optionally also to an orientation. The system further comprises a computational system, which has an input interface for receiving said imaging data, and an output interface for outputting data for controlling the movement of the positioning device. The computational system is configured to determine a position of the at least one landmark based on the imaging data, and to control the position of the positioning device with respect to the at least one target zone based on the determined position of the at least one landmark.

The at least one target zone may be a region on the patient where the medical module is to be positioned or should be targeted. As an example, the at least one target zone could be a region or point that is located over a blood vessel on the skin of the patient. The at least one target zone may comprise several target zones located on the patient.

The at least one landmark may be a point or region of reference. The at least one landmark may be used as an origin for locating other points of interest and in particular the at least one target zone. The at least one landmark may also be used as a reference point with known coordinates and optionally also with an encoded orientation, allowing to determine a coordinate system from the at least one landmark. The at least one landmark may be an optically recognizable symbol or an optically recognizable structure.

The patient may be fixed on a surgical table by a body holding structure, for example an open receptacle for receiving and fixing the patient's leg to the surgical table. The body holding structure may be used as the at least one landmark.

The at least one landmark facilitates the determination of a position or a reference system, namely the position of the at least one target zone or a reference system for the at least one target zone. The at least one landmark helps to determine a position or a reference system in a repeatable way that is not prone to errors, especially not to human errors.

The imaging means may, for example, be a digital camera, an imaging sensor like a charge-coupled device (CCD) or an active-pixel sensor (CMOS sensor) with a lens system, a video camera, a stereoscopic camera setup, a digital camera together with light detection and ranging (LIDAR), or a digital camera together with an infrared (IR) depth camera. The at least one imaging probe may, for example, be positioned on the positioning device or be positioned in a defined spatial relationship to the positioning device, preferentially less than 20 meters away from the positioning device, more preferentially less than 5 meters away from the positioning device.

The at least one landmark is arranged in particular in a predefined distance to the at least one target zone. The predefined relationship at least allows to determine, based on the position of the at -least one landmark, a position of the at least one target zone. Preferably, the predefined relationship further allows to determine the orientation of the at least one target zone given the orientation of the at least one landmark. The predefined relationship may allow to determine a coordinate reference system of the at least one target zone based on a coordinate reference system of the at least one landmark.

The imaging means may acquire imaging data from a region where the patient is laying, for example the region above a surgical table on which.the patient may be laying. If the head of the patient can be imaged by the imaging means, for example if the head of the patient is not covered by a surgical drape, the distance between the head and the at least one target zone may be measured from pre-operative computed tomography scans (CT scans). This is an addition to the at least one landmark and not an actual landmark. The distance between the head and the at least one target zone may be used by the computational system in order to move the positioning device from the head of the patient to the at least one target zone, whereas at this region a search for the at least one landmark may be done for more precise localization of the at least one target zone.

The computational system may use the imaging data to make body skin estimations from shape analysis. The computational system may predict the position of the at least one target zone from the shape analysis of the skin surface based on the imaging data.

In a preferred embodiment, the system further comprises a medical module, which comprises at least a surgical instrument. The surgical instrument is attachable or attached to the mount for a medical module.

The at least one surgical tool may, for example, be a catheter needle, an injection needle, a dilator, a speculum, an endoscope as for instance a fiber optic endoscope, a surgical scissor, or a mechanical cutter like a scalpel.

In an advantageous embodiment, the medical module further comprises at least one imaging probe, in particular an ultrasound probe, for imaging the application site of the probe.

The at least one imaging probe may, for example, be an ultrasound probe, and/or a photoacoustic imaging probe, and/or a pulsed infrared probe. Preferably, the at least one imaging probe may be used to detect blood volume changes in the microvascular bed of tissue, allowing to obtain a photoplethysmogram.

Using such a probe within the target zone allows to create an image of internal body structures of a patient,- for example
blood vessels. It is particularly advantageous to use an ultrasound probe to localize blood vessels in a patient and to determine their position in the patient, for example with respect to the ultrasound probe. Knowing internal body structures allows, for example, to control the positioning device such as to precisely position the at least one surgical tool over internal body structures like blood vessels.

The at least one imaging probe may be used to refine the position (increase the precision of the determined position), preferably also or only the orientation, of the at least one target zone that was determined by the computational system based on the at least one landmark. This may be done, for example, by comparing pre-operative computed tomography scans (CT scans) with images recorded by the imaging probe.

Red or infrared light, preferably pulsed, may be projected on the body surface of the patient in order to detect change in blood flow during heart beats as a change in the amount of light transmitted through the body of the patient or as a change in the amount of light reflected on the body of the patient, whereas in the latter case the change may come from the light reflected on the blood vessels of the patient.

Preferentially, the medical module may also comprise an additional medical imaging device instrument or a robotic device which may be configured to generate 3D or higher dimensional medical imaging data.

The additional device may be, for example, a 3D ultrasound probe.

In a particularly preferred embodiment the at least one landmark is formed by a boundary delimiting the at least one target zone. Preferably, the computational system of the system is configured to determine the shape of said boundary delimiting the at least one target zone.

The patient is preferably covered by a surgical drape. Preferably, the at least one landmark is, for example, an incised area of the surgical drape covering the patient. The incision forms a boundary, which preferentially delimits the at least one target zone. The incision can, for example, have a geometric form like a square, a rectangle, a triangle, a polygon, or a circle, and the area of the incision is preferentially smaller than 2500 cm2, more preferentially smaller than 500 cm2.

The computational system may be used to identify the patient position and/or orientation on the surgical table using, for example, a trained algorithm. For the same purpose, a transparent surgical drape or a thermal camera may be used, whereas the thermal camera may be able to obtain thermal imaging data of the patient even when the patient is covered by the surgical drape. The thermal imaging data may be used by the computational system to determine the orientation of the patient covered by the surgical drape.

Determining the shape of the at least one landmark is advantageous as it allows, for example, to extract more information from the at least one landmark. Preferentially, the shape of the at least one landmark is such that it is non-symmetric at least in one direction, therefore allowing to use the orientation of the at least one landmark to indicate an orientation of the patient. As an example, an incision in the surgical drape having a shape of an isosceles triangle with a vertical axis of symmetry, may be used as the at least one landmark. The vertical axis of the isosceles triangle is used as an arrow and the surgical drape is placed over the patient in such a way that the arrow points to the head of the patient, therefore defining the orientation in addition to position.

In addition or as an alternative to the orientation information of the patient from the at least one landmark, information on the orientation of the patient may be indicated by symbols, for example the patient may wear a headband comprising a distinctive sign like a predefined colour or an infrared reflecting coating. In this case, the headband is not covered by the surgical drape or is still visible through the surgical drape. The symbols may be recognized on the imaging data by the computational system which may associate it to a predefined location on the patient, for example the head of the patient. Combining this information with the position data of the at least one landmark, may allow to determine the orientation of the patient.

The face of the patient may be used to detect the patient orientation in that it may allow to distinguish the left and right.

For determining the orientation and/or the position of the patient with respect to, for example, the surgical table, a deformable surface, preferably a vacuum positioning pillow, may be placed between the patient and the surface of the surgical table. There may be means rigidly attached to the deformable surface to quantify the occurring contact between the patient and surface when placed upon the deformable surface, further referred to as body contact sensor strips. These may comprise:
- Strain or stretch sensors, which may rely on resistance change, for example strain gauges or conductive rubber, and/or they may rely on capacitive change, for example dielectric dimensional change, and/or they may rely on piezoelectric charge in function of strain. Advantageously the strain or stretch sensors may be suited for integration into or on textiles, such as for example for integration into or on the surgical drape.
- Contact sensors for distinguishing contact with the patient. The contact may be detected, for instance, through electrical conductivity, for this, a conductive filament, for example a silver filament, may be integrated into a textile on top of, for example, the surgical table.
- Force or pressure sensors; which may be derived from the strain sensors in combination with a known flexure.
- Shape sensors; which may be derived from embedding discrete grids of the strain or stretch sensors, or the contact sensors, or the force or pressure sensors. Alternatively or in addition, dedicated shape sensors such as tower-grating FBG or 3D tracking sensors, such as electromagnetic tracking, may be embedded.
- Distance sensors; which may be used for measurement of distance from the top pillow surface with respect to the surgical table. For instance, a grid of optical distance sensors can be used, which may measure distances along the normal with respect to the surgical table surface.

The deformable surface may enable at least a partial quantification of the patient reference with respect to a positional frame, wherein the reference frame may, for example, be the reference frame of the positioning device. This may, for example, be based on one of the following:
- Patient body orientation with respect to the table, wherein there may be the possibility to determine the direction of the superior/inferior axis of the patient body on the table, assuming the patient is lying on their back. The superior axis may be the one towards the head of the patient and the inferior axis may be the one towards the feet of the patient.
- Patient body contour with respect to the table
- Registration of a digital anatomy model to the surgical table. This may be done by applying a registration method using an in-situ anatomy quantification and the digital model as inputs, and providing pose information for the digital model with respect to the surgical table.

In a preferred embodiment, the positioning device has a robotic arm.

The robotic arm is preferentially configured to execute positioning tasks with a positioning accuracy of better than 20 millimeters, preferentially better than 5 millimeters, more preferentially better than 1 millimeter. The robotic arm preferentially has a movement range of less than 5 meters, more preferentially less than 3 meters, most preferably less than 1 meter. The robotic arm may be configured to make high speed movements, for example more than 5 centimeters per second, with a lower accuracy than low speed movements, for example less than 5 centimeter per second.

In a preferred embodiment, the imaging means is configured to provide depth information of objects in a field of view of the imaging means.

A field of view of the imaging means is a solid angle through which the imaging means, for example a camera or an infrared camera, is sensitive to electromagnetic radiation. If the imaging means comprises more than one image acquisition units, for example if it comprises a 2D camera and an IR depth camera, or if it comprises a 2D camera and a LIDAR, the imaging means has more than one field of view as each image acquisition unit will have its own field of view.

Depth information is information that at least allows to extract distances between objects, preferentially also allows to extract spatial information such as relative position between objects, for example allowing to determine an Euclidian vector specifying the position of one object relative to the other object, or absolute position of objects in a given reference coordinate system.

It may be that not all image acquisition units comprised in the imaging means can be used to determine depth information. It may be, that the combination of two image acquisition units allows to obtain depth information, whereas taking each one of the two optical instruments individually does not allow to extract depth information. For example, two cameras that are horizontally displaced from one another may be used to obtain two differing images of a scene taken under a different angle. By comparing these two images, depth information can be obtained.

Since the imaging means relies on discrete imaging sensors, for example a sensor with a given number of pixels, like a charge coupled device (CCD), the depth information will also be discrete.

In a particularly preferred embodiment, the imaging means comprises at least a stereoscopic camera setup, a 2D camera and a LIDAR, or a 2D camera and an IR depth camera.

A stereoscopic camera setup may be a camera with two or more lenses with a separate image sensor or film frame for each lens, or it may be a setup composed of two or more individual cameras. For a setup composed of two or more individual cameras, the cameras have to be triggered at sensibly the same time, i.e. the cameras each acquire an image at sensibly the same time. Sensibly the same time refers to a time difference of less than 1 second, preferably less than 0.1 second, more preferably less than 0.01 second.

The computational system may further be configured to perform image registration between preoperative images such as, for example, between 2D pelvis fluoroscopy and 3D CT scans or between 3D CT body surface and 3D depth camera, in order to determine the position of the at least one target zone.

It is particularly preferred that the imaging means may comprise a rotating 3D LIDAR or a fisheye RGB camera. If the imaging means comprises a rotating 3D LIDAR or a fisheye RGB camera, the computational system may determine with high accuracy obstacles surrounding the patient and/or the positioning system.

In a preferred embodiment, the computational system is configured to identify, in real time, the position of the at least one landmark based on the imaging data.

The computational system may use the real time position of the at least one landmark to adjust the position of the positioning device in respect to movements of the patient. Movements of the at least one landmark and/or the at least one target zone, may be due, as an example, to breathing of the patient.

Identification in real time of the position of the at least one landmark means that the position of the at least one landmark is identified repeatedly, preferably continuously. A small time-delay, for example less than 3 seconds, preferably less than 1 second, in a repeated, preferably continuous, identification of the position of the at least one landmark is still considered a real time identification.

Identification in real time of the position of the at least one landmark may be done by means of conventional image processing or preferably with artificial intelligence. As an example, a neural network trained on imaging data with or without depth information may be used for identification in real time of the position of the at least one landmark. The same method can be used in both above cases. A possible deep neural network (DNN) model is an encoder-decoder architecture. The encoder allows to extract robust features of imaging data, respectively features of imaging data comprising depth information, and can either be a backbone pretrained on a public image dataset, for example a MobileNet backbone, or trained from scratch using a large number of imaging data of the imaging means, respectively imaging data comprising depth information of the imaging means, or comparable imaging data, which may, for example, be taken by another imaging means and/or comprise a different type of landmark. The decoder projects the extracted features back onto the original image size through, for example, a series of upsampling layers, in particular, the last layer performs operative field semantic segmentation by classifying each pixel either as landmark or background. Additional image post-processing, aiming for instance to filter out spurious detections, may be applied. The at least one landmark can be extracted by means of the connected components algorithm.

In an advantageous embodiment, the computational system is configured to further decode fiducial markers that are preferably placed in proximity of the at least one target zone.

Fiducial markers may be used as landmarks or in combination to another landmark.

In proximity of the at least one target zone is, for example, closer than 30 centimeters, preferably closer than 20 centimeters, more preferably closer than 5 centimeters to the at least one target zone.

Information relating to the position of the fiducial markers and/or the position of at least one of the at least one landmark can be encoded in the fiducial markers. The position of the at least one landmark may, for example, for a landmark that has a geometrical form of a square, be given by specifying the coordinates of the four corners of the landmark with respect to the given fiducial markers. Encoded in the fiducial markers is, preferably, an identification for the at least one landmark, for example specifying that the landmark just next to a given one of the fiducial markers is the landmark "A", or any distinctive identification, preferably, the total number of landmarks may also be encoded in the fiducial markers.

Identification in real time of the position of the at least one landmark may be done by means of decoding, in real time, by the computational system, the position encoded in the fiducial markers.

Fiducial markers, for example AprilTags, ArUco tags, ARTags, AR-ToolKit tags, or QR codes, placed in proximity of the at least one target zone, may be fixed, for example, to the surgical drape, directly to the patient, or to any suitable object in proximity of the at least one target zone. As an example, fiducial markers can be directly knitted, glued, or mechanically attached to the surgical drapes, preferably around openings in the surgical drapes, whereas the openings may be used as landmarks. Ad hoc devices might also be used to place and remove fiducial markers fixed to the patient body.

Fiducial markers may also be used to store in information like patient orientation or general patient information. Fiducial markers that only store information like patient orientation or general patient information may not need to be placed in proximity of the at least one target zone.

In a particularly preferred embodiment, the computational system is configured to determine a centroid of the at least one landmark from the imaging data, and to determine 3D Cartesian coordinates of points in the imaging data, and to determine a 3D surface normal at the 3D centroid of the at least one landmark based on the 3D Cartesian coordinates of points in proximity of the centroid.

The 3D centroid, also commonly referred to as geometric center, of the at least one landmark is the point on which the at least one landmark would balance if it were placed on a needle.

Imaging data are discrete data. For example, imaging data of a landmark will comprise a given number of pixels, for example 2 Megapixels.

The 3D Cartesian coordinates of the points lying in a small rectangular region, preferably 50x50 pixels, more preferably 30x30 pixels, most preferably 10x10 pixels around the centroid of the at least one landmark, are used to robustly estimate the 3D surface normal at the 3D centroid of the at least one landmark. This can be achieved, for instance, by fitting a tangent plane to the surface in the vicinity of the 3D centroid using a set of 3D neighbouring points by means of a least squared optimization technique, and deriving the surface normal directly from the so estimated plane equation.

Preferably, the computational system is configured to determine a centroid of the at least one landmark based on the imaging data. Each one of the at least one landmark has its own associated centroid.

In a preferred embodiment, the computational system is configured to fit the shape of the at least one landmark.

Fitting the shape of the at least one landmark may be done by determining, based on the imaging data, the contour of the at least one landmark which can, for example, have the shape of a geometric form like a square, a rectangle, a triangle, a polygon, or a circle, followed by fitting a geometrical form that minimizes the differences between the fitted geometrical form and the determined contour of the at least one landmark. The geometric figure does not need to lay in a 2 dimensional plane, it can, for example, have a curvature or be bent and curved in different directions at different locations of the geometric figure. Therefore, the shape of the at least one landmark can be accurately fitted even if the at least one landmark has a non 2 dimensional shape.

For example a 3D rectangular shape of the at least one landmark may be directly obtained from the four 3D coordinates of the corner points of the 2D fitting rectangle of the extracted at least one landmark if the shape of the landmark is known.

In a preferred embodiment, the computational system is configured to further determine a relative orientation and/or position between the mount for a medical module and the at least one landmark based on the imaging data.

Knowing the relative orientation between the mount for a medical module and the at least one landmark allows, for example,
- that the mount for a medical module can be placed by the positioning device at a given orientation relative to the at least one landmark;
- to position a medical instrument mounted on the mount for a medical module, with a relative orientation with respect to the at least one landmark.
As the at least one landmark is arranged in a predefined relationship to the at least one target zone, the medical instrument can be positioned with a predefined relative orientation to the at least one target zone. Knowing this relative orientation helps placing, for example, a medical instrument comprised in the medical module with a desired orientation.

The pose of the medical module (i.e. a combination of position and orientation) can be arbitrarily chosen. However, for some applications it is important to place the medical module, preferably the medical instrument, at a predefined angle to an axis, preferably to the normal of the target zone. As an example, the longitudinal probe axis, i.e. the axis along the imaging probe, is desired to be aligned with the normal to the target zone. In such case, the medical module orientation can be selected to ensure an orthogonal alignment between the imaging probe and the determined surface of the at least target zone. A coordinate reference system may be obtained, for example, by defining a second axis such that it follows, for example, the patient orientation. The third axis needed to obtain a 3D coordinate reference system would need to be chosen orthogonal to both of the axes just described.

The patient orientation may be determined by using the ultrasound probe of the at least one imaging probe and by using the Doppler effect. As an example, artificial intelligence may be trained to detect and separate vein from artery flow based on imaging data of the ultrasound probe. By comparing the obtained results with known anatomy of the human body the orientation of the patient may be determined.

In an advantageous embodiment, the computational system is configured to determine a 3D map of the surroundings of the at least one landmark based on the depth information of objects in the field of view of the imaging means.

In a preferred embodiment, the computational system is configured to determine, based on the 3D map of the surroundings, a path free from obstacles connecting the current position of the mount with the medical device and a target position close to the at least one target zone. The mount is movable along the path without colliding with an obstacle.

According to another aspect of the invention, a computational system for determining positioning data based on imaging data is disclosed. The computational system comprises a data input for receiving imaging data, and a data output for outputting positioning data. The computational system is configured to determine a position of at least one landmark with respect to a reference, based on received imaging data. The computational system is configured to provide positioning data based on the determined position of the at least one landmark, and to output the positioning data.

The computational system for determining positioning data based on imaging data can be used in relation with the system for positioning a medical device with respect to at least one target zone of patient as previously described. The computational system may be arranged at a remote location from the positioning device of the system for positioning a medical device.

According to still another aspect of the invention, a method for operating a system for positioning a medical device with respect to a target zone position, preferably using the system for positioning a medical device with respect to a target zone as previously described. The system for positioning the medical device comprises a mount for a medical module, and a positioning device for moving with respect to the at least one target zone, and an imaging means, and a computational system. The computational system comprises an input interface for receiving said imaging data, and an output interface for outputting data for controlling the movement of the positioning device. Imaging data are obtained with the imaging means. A position of the at least one landmark is determined, based on the imaging data, by the computational system. The position of the positioning device is controlled with respect to the at least one target zone, based on the determined position of the at least one landmark, by the computational system.

According to still another aspect of the invention, a computer program product is provided. The computer program product has program code instructions stored on a computer readable medium to execute the method steps of the method for operating a system for positioning a module with respect to a target zone when said program is executed on a computer.

According to still another aspect of the invention, a non-transient computer readable storage medium storing the computer program product is disclosed.

The invention is now described with reference to certain embodiments and figures which show:
- Figure 1:: Schematic representation of a first embodiment of a system for positioning a medical device with respect to at least one target zone of a patient;
- Figure 2:: Computed poses of two landmarks and their computed centroid, for an embodiment of a landmark.
- Figure 3:: A representation of an embodiment of a system for patient body orientation detection.
- Figure 4:: A representation of an embodiment of a system for patient body orientation detection using image registration

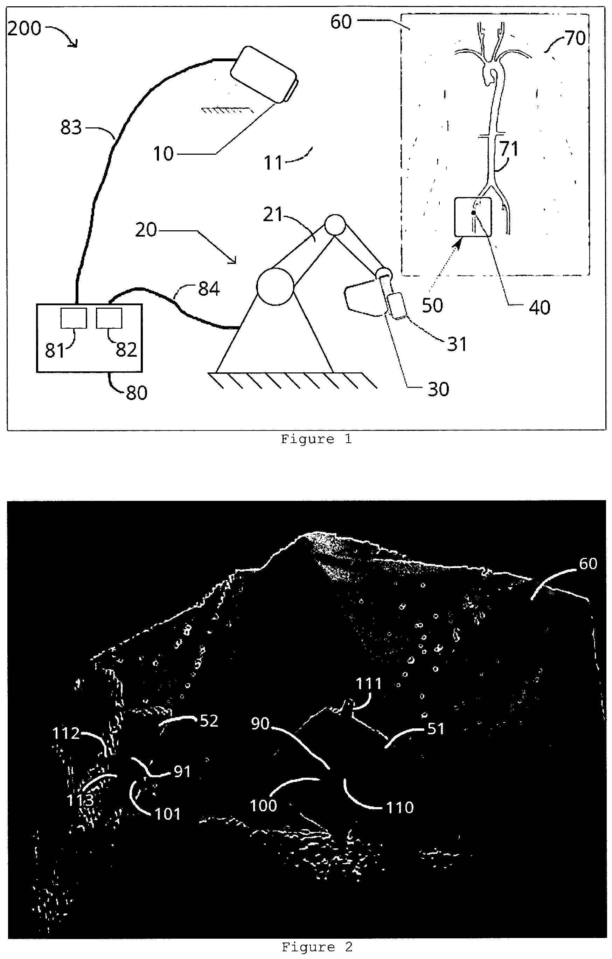

Figure 1 shows a first embodiment of a system 200. The system 200 comprises a positioning device 20. The positioning device 20 is configured to move a mount 30 for a medical module 31 with respect to a target zone 40 on a patient 70 covered by a surgical drape 60. The target zone 40 is an area neighboring a blood vessel 71 of the patient 70. An imaging means 10, with its field of view 11, is configured to obtain imaging data of a landmark 50 arranged in a predefined relationship to the target zone 40. The landmark is formed by an opening in the surgical drape 60. The imaging means 10 is connected to a computational system 80 via a connection 83 (wired or wireless). The computational system 80 comprises an input interface 81 for receiving said imaging data. The computational system 80 is configured to determine a position of the landmark based on the imaging data. The computational system 80 is configured to control the position of the positioning device 20 with respect to the target zone 40 by outputting data through an output interface 82, which is comprised in the computational system 80. The output data are used for controlling the movement of the positioning device 20 that is connected to the computational system 80 via a connection 84 (wired or wireless). The positioning device 20 comprises a robotic arm 21 that allows for precise positioning of the mount 30 for the medical module 31.

Figure 2: schematic of an experimental result showing poses, i.e. positions and orientations, of two landmarks and their centroid. Both, the poses and the centroids, were determined by the computational system 80. A surgical drape 60 is covering a patient 70. The surgical drape 60 has two openings that are used as landmarks 51, 52. The landmarks 51, 52 both have their centroid 90, 91 determined by the computational system 80. Based on imaging data comprising depth information, the computational system can determine:
- A normal 100, 101 to a local surface of the patient 70 at the centroid 90, 91 of the landmark 52, 51. The normal 100, 101 extends from the centroid 90, 91 towards inside the patient 70.
- A first tangent 110, 112 to surface at centroid which forms a right angle with the normal 100, 101 to the surface of the patient 70 at the centroid 90, 91 of the landmark 52, 51.
- A second tangent 111, 113 to surface at centroid, which forms a right angle with the normal 100, 101 to the surface of the patient 70 at the centroid 90, 91 of the landmark 52, 51 and which also forms a right angle with the first tangent 110, 112 to surface at centroid.

The normal 100, the first tangent 110 and the second tangent 111 form an orthogonal basis. The normal 101, the first tangent 112 and the second tangent 113 also form an orthogonal basis. Preferably, the normal 100, 101, the first tangent 110, 112 to surface at centroid and the second tangent 111, 113 to surface at centroid are determined by the computational system such that they are all of unitary length. Therefore, they form two orthonormal bases.

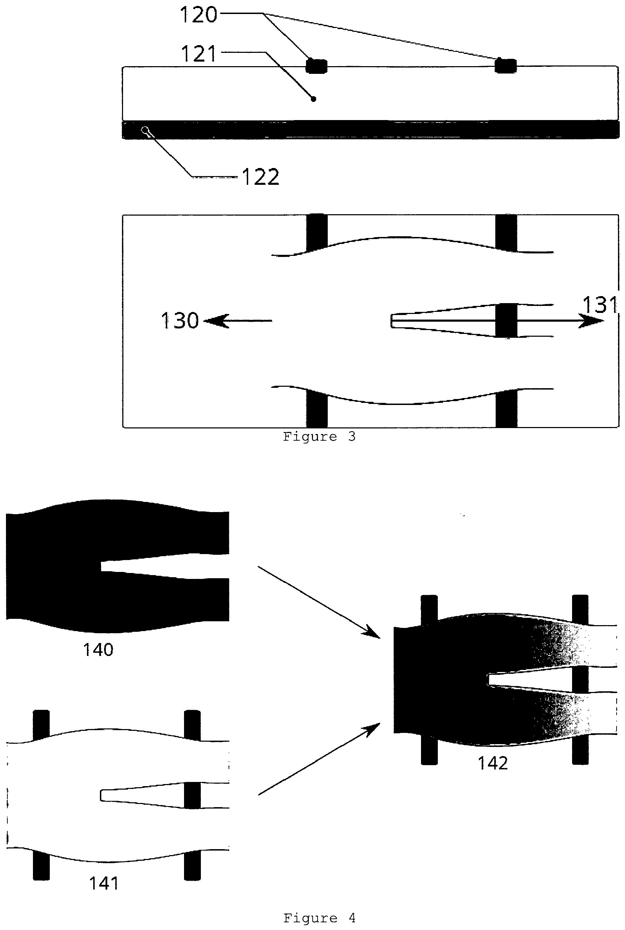

Figure 3 shows an embodiment of a surgical table usable in the system as shown in Figure 1. The surgical table surface 122 is covered by a vacuum positioning pillow 121. On top of the vacuum positioning pillow 121, there are sensors 120, for example contact sensors, preferably contact sensor strips. Based on information from the vacuum positioning pillow 121 and/or the sensors 120, the computational system 80 may determine the orientation of the patient, who is lying on the vacuum positioning pillow 121 and covering at least part of the sensors 120. Based on information from the vacuum positioning pillow 121 and/or the sensors 120, the computational system 80 may determine the direction of the superior axis 130 of the patient and/or the direction of the inferior axis 131 of the patient.

Figure 4 shows an embodiment wherein image registration is performed, by the computational system 80, between a digital model 140 and an in-situ anatomy quantification 141. Based on the image registration, pose information for the digital model 142 with respect to the surgical table is obtained. The in-situ anatomy quantification 141 may be determined by the computational system 80 based on a deformable surface, preferably a vacuum positioning pillow, that is placed between the patient and the surgical table surface 122. The digital model may be determined by the computational system 80 based on the imaging data, preferably based on the imaging data including depth information, more preferably based on preoperative 3D CT scans.

## Claims

1. A system (200) for positioning a medical device with respect to at least one target zone (40) of patient (70), the system comprising:
- a mount (30) for a medical module (31), and
- a positioning device (20) configured to move the mount (30) for a medical module (31) with respect to the at least one target zone (40), and
- an imaging means (10), configured to at least obtain imaging data of at least one landmark (50, 51, 52) arranged in a predefined relationship to the at least one target zone (40), and
- a computational system (80)
wherein the computational system (80) comprises:
- an input interface (81) for receiving said imaging data, and
- an output interface (82) for outputting data for controlling the movement of the positioning device (20)
wherein the computational system (80) is configured:
- to determine a position of the at least one landmark (50, 51, 52) based on the imaging data, and
- to control the position of the positioning device (20) with respect to the at least one target zone (40) based on the determined position of the at least one landmark (50, 51, 52).

2. The system according to claim 1, further comprising a medical module (31), which comprises at least a surgical instrument, that is attachable or attached to the mount (30) for a medical module (31).

3. The system according to claim 2, wherein the medical module (31) further comprises at least one imaging probe, in particular an ultrasound probe, for imaging the application site of the probe.

4. The system according to one of claims 1 to 3, wherein the computational system (80) is further configured to determine the shape of the landmark (50, 51, 52) formed by a boundary delimiting the target zone (40).

5. The system according to one of claims 1 to 4, wherein the positioning device (20) has a robotic arm (21).

6. The system according to one of claims 1 to 5, wherein the imaging means (10) is configured to provide depth information of objects in a field of view (11) of the imaging means (10).

7. The system according to one of claims 1 to 6, wherein the imaging means (10) comprises at least one of
- a stereoscopic camera setup
- a 2D camera and a LIDAR, and
- a 2D camera and an IR depth camera.

8. The system according to one of claims 1 to 7, wherein the computational system (80) is configured to identify the position of the at least one landmark (50, 51, 52) based on the imaging data in real-time.

9. The system according to one of claims 1 to 8, wherein the computational system (80) is configured to decode fiducial markers that are preferably placed in proximity of the at least one target zone (40).

10. The system according to one of claims 1 to 9, wherein the computational system (80) is configured
- to determine a centroid (90, 91) of the at least one landmark (50, 51, 52) from the imaging data,
- to determine 3D Cartesian coordinates of points in the imaging data, and
- to determine a 3D surface normal (100, 101) at the 3D centroid of the at least one landmark (50, 51, 52) based on the 3D Cartesian coordinates of points in proximity of the centroid (90, 91).

11. The system according to one of claims 1 to 10, wherein the computational system (80) is configured to fit the shape of the at least one landmark (50, 51, 52).

12. The system according to one of claims 1 to 11, wherein the computational system (80) is configured to determine a relative orientation between the mount (30) for a medical module (31) and the at least one landmark (50, 51, 52).

13. The system according to one of claims 6 to 12, wherein the computational system (80) is configured to determine a 3D map of a surroundings of the at least one landmark (50, 51, 52) based on the depth information of objects in the field of view (11) of the imaging means (10).

14. The system according to claim 13, wherein the computational system (80) is configured to determine, based on the 3D map of the surroundings, a path free from obstacles connecting the current position of the mount (30) for a medical module (31) and the target zone (40), the mount (30) for a medical module (31), preferably comprising the medical module (31), being movable along the path free from obstacles without colliding with an obstacle.

15. A computational system (80) for determining positioning data based on imaging data, the system comprising:
- an input interface (81) for receiving imaging data, and
- an output interface (82) for outputting positioning data,
wherein the computational system (80) is configured:
- to determine a position of at least one landmark (50, 51, 52) with respect to a reference, based on received imaging data,
- to provide positioning data based on the determined position of the at least one landmark (50, 51, 52), and
- to output the determined landmark (50, 51, 52) position and/or the positioning data.

16. Method for operating a system for positioning a medical device with respect to a target zone (40), preferably using the system (200) for positioning a medical device with respect to a target zone (40) according to one of claims 1 to 14, wherein the system comprises:
- a mount (30) for a medical module (31), and
- a positioning device (20) for moving the mount (30) for the medical module (31) with respect to the at least one target zone (40), and
- an imaging means (10), and
- a computational system (80)
wherein the computational system (80) comprises:
- an input interface (81) for receiving said imaging data, and
- an output interface (82) for outputting data for controlling the movement of the positioning device (20),
wherein the method composes the steps of
- obtaining imaging data, with the imaging means (10), of at least one landmark (50, 51, 52) arranged in a predefined relationship to the at least one target zone (40),
- determining a position of the at least one landmark (50, 51, 52), based on the imaging data, by the computational system (80), and
- controlling the position of the positioning device (20) with respect to the at least one target zone (40), based on the determined position of the at least one landmark (50, 51, 52), by the computational system (80).

17. A computer program product comprising program code instructions stored on a computer-readable medium to execute the method steps according to claim 16 when said program is executed on a computer

18. A non-transient computer readable storage medium storing a computer program product according to claim 17.
